(19) **Europäisches Patentamt**
**European Patent Office**
**Office européen des brevets**

(11) **EP 1 358 357 B1**

(12) **EUROPEAN PATENT SPECIFICATION**

(45) Date of publication and mention
of the grant of the patent:
**09.03.2011 Bulletin 2011/10**

(51) Int Cl.:
*C12Q 1/68* (2006.01)   *G01N 33/543* (2006.01)
*G01T 1/164* (2006.01)

(21) Application number: **02716498.7**

(22) Date of filing: **11.01.2002**

(86) International application number:
**PCT/NO2002/000014**

(87) International publication number:
**WO 2002/059365 (01.08.2002 Gazette 2002/31)**

(54) **METHOD AND APPARATUS FOR SIMULTANEOUS QUANTIFICATION OF DIFFERENT RADIONUCLIDES ON THE SURFACE OF A BIOLOGICAL MICROARRAY**

VERFAHREN UND VORRICHTUNG ZUR GLEICHZEITIGEN QUANTIFIZIERUNG VERSCHIEDENER RADIONUKLIDE AUF DER OBERFLÄCHE EINES BIOLOGISCHEN MIKROARRAYS

PROCEDE ET APPAREIL DE QUANTIFICATION SIMULTANEE DE DIFFERENTS RADIONUCLEIDES A LA SURFACE D'UN MICRORESEAU BIOLOGIQUE

(84) Designated Contracting States:
**AT BE CH CY DE DK ES FI FR GB GR IE IT LI LU MC NL PT SE TR**

(30) Priority: **12.01.2001 NO 20010229**

(43) Date of publication of application:
**05.11.2003 Bulletin 2003/45**

(73) Proprietor: **Biomolex AS**
**0319 Oslo (NO)**

(72) Inventors:
• **HOVIG, Eivind**
  **N-0851 OSLO (NO)**
• **SKRETTING, Arne**
  **N-1341 SLEPENDEN (NO)**
• **NYGARD, Einar**
  **N-1386 ASKER (NO)**

• **KVINNSLAND, Yngve**
  **N-5225 Nesttun (NO)**
• **BREISTOL, Knut**
  **N-3121 Tonsberg (NO)**
• **YOSHIOKA, Koki**
  **N-0188 OSLO (NO)**

(74) Representative: **Tombling, Adrian George et al Withers & Rogers LLP**
**Goldings House**
**2 Hays Lane**
**London**
**SE1 2HW (GB)**

(56) References cited:
**EP-A2- 0 268 406    EP-A2- 0 853 427**
**WO-A1-99/08233    GB-A- 2 311 198**
**US-A- 4 967 084    US-A- 5 117 114**
**US-A- 5 656 818    US-A- 5 866 907**

## Description

### Technical field

The present invention relates to a method and apparatus for simultaneous quantification of the amounts of one or more radioactive nuclides within arbitrary regions on a surface where these nuclides have been deposited, adsorbed or fixed. These radioactive nuclides serve as markers on compounds that typically have been incorporated into tissue sections or into larger biological molecules that by various mechanisms have been bound to chemical substances on this surface. The method is especially well suited for DNA microarray deductions through the use of nucleotides labelled with different beta-emitting radionuclides.

### Background

It is widely believed that thousands of genes and their products, i.e. RNA and proteins, in a living organism function in a complicated and orchestrated way that creates the mystery of life. However, traditional methods in molecular biology generally work on a "one gene in one experiment" basis, which makes it hard to achieve the overall picture of the gene function. Thus, biological microarrays represents one of the most potent new tools in biological research to emerge in recent years, since it gives the opportunity to study a complete set of genes and their products simultaneously.

A microarray is an ordered arrangement of biological molecules immobilised in sample spots on a test plate which provides a medium for matching known and unknown samples of biological molecules. The immobilised molecules on the test plate are often denoted probe molecules, while the biological molecules from the test samples are denoted target molecules. In the case when the probe molecules and target molecules forms specific complementary pairs of biological molecules, the ordered arrangement of the test spots can be employed to identify specific biological molecules in a test sample from an organism and also to determine the abundance of these molecules. Typical examples of complementary biological molecules are hybridisation pairs of DNA, gene-anti gene etc. In microarrays the sample spots are typically less than 200 $\mu$m in diameter, but "macroarrays" with sample spots with diameters of typically 300 $\mu$m or larger have been described.

The biological microarray technique can be applied for numerous applications such as diagnosis, identification/ discovery of new genes and proteins, drug discovery, pharmacological and toxicological research etc. The technique can also be employed to comparison tests where biological components from several sources are adsorbed onto the same array, for instance from a healthy cell and a tumour cell.

Among biological microarrays, it is especially one type that has drawn attention the latest years; the DNA-microarray. This technology promises to monitor the whole genome on a single chip, and thereby make it possible to acquire a better picture of the interactions between thousands of genes simultaneously.

### Prior art

In general, the conventional method of determining biological activity by DNA-microarrays can be described as follows; strands of probe cDNA (typically 500-5000 bases long) are immobilised in a specific and ordered array onto a solid plate (typically a glass plate). Then the probe cDNA is exposed to one or several targets (marked cDNA from the test samples) either separately or in a mixture. The targets, labelled cDNA, are produced enzymatically by reverse transcriptase from samples of RNA which are extracted from the test samples and labelled with specific marker molecules. The reverse-transcribed RNA transcripts of the samples are hybridised with the probe cDNA on the microarray. Thus the amount and type of each target cDNA can be determined by measuring the location and concentration of each marker molecule at each test spot on the microarray, since the marker signal from each test spot reflects the relative transcript amounts for each specific transcript at each test spot on the microarray. To eliminate sample variation, the signal ratio between two competing samples is the preferred measurement.

#### Fluorescence tagged nucleotides

Traditionally, the detection of signals using this technology has been based on *in vitro* incorporated nucleotides labelled with suitable fluorophores, that is, specific fluorophores (fluorescence molecules) of a distinctive colour are inserted into the RNA extracted from each sample, respectively. Thus nucleotides labelled with fluorophores of a distinctive colour are incorporated into the target cDNA which will be hybridised to the probe cDNA. The fluorophores may be excited by different wavelengths, and similarly also emit at different wavelengths. Laser light and the use of appropriate filters to separate signals from two or more cDNA populations will generally achieve this.

However, the general need for starting material is in the range of 50$\mu$g of total RNA, or approximately 5 x $10^7$ cell equivalents. This relatively high amount of material excludes the use of standard technology from a number of very relevant applications, including clinical diagnostics. One highly significant factor in this lack of sensitivity is the low incorporation rate of current fluorophore-tagged nucleotides in the reverse transcription of cDNA from RNA. Thus only a relatively low number of fluorescence molecules will be incorporated per synthesised cDNA. Also, applications requiring incorporation of fluorescence through cell culture will be excluded, as fluorophore-tagged nucleotides will generally not be included through the cellular machinery. Emerging techniques to achieve better signal strength include enzymatic amplification of sample material and chemical signal amplification. Such methods demonstrate that it is possible to reduce sample size.

Amplification techniques have recently been published allowing a reduction in sample size down to 100 ng total RNA starting material [1]. This is achieved through one or more rounds of cycling between RNA and cDNA. In this reaction it is possible in each round to obtain approximately a 50-fold increase of the material by attaching a T7 promoter at one end to enable generation of RNA, and at the other end exploiting a feature of certain reverse transcriptases to add a specific primer to all cDNAs at most 5' end at the time of first reverse transcription. This feature is necessary to avoid generation of shorter length cDNAs.

An alternative strategy is to amplify the signal from the test material through chemical means. The most sensitive strategy so far available relies on nested tangles of labelled branched synthetic DNA molecules [2, 3, 4]. These may be bound to poly-A tails of cDNA prior to array hybridisation. Generally, a 250-fold increase in signal strength may be achieved.

Neither of these strategies have been rigorously tested for reliable performance and sensitivity levels. It is likely that amplification techniques will lead to degrees of bias of the starting material, due to the enzymatic nature of the process combined with the large variation in mRNA length for different transcripts.

The large amount of test material necessary to achieve adequate signal strength, and the problem that the available fluorophores are accepted only with difficulty by the reverse transcriptase enzyme represents thus considerable disadvantages in the prior art.

Radioactivity labelled nucleotides

It is known that the problem with low acceptance by the reverse transcriptase enzyme can be solved by employing radioactive isotopes for the labelling of nucleotides.

Historically, radioactive isotopes have been in widespread use for sensitive detection and quantification of nucleic acids. The common use has been confined to the use of one single, usually beta-emitting, radionuclide, incorporated either into a probe (detector nucleic acid) or directly in vivo. The detection has been performed using liquid scintillation or gamma counters, and in the case of a two-dimensional distribution (e.g. Northern and Southern blots), autoradiography film, phosphor imagers, and digital autoradiography systems has been employed.

Measurements of the distribution of a radioactive compound in a thin section of tissue or Southern and Northern nucleic acid blots has traditionally been performed with film autoradiography, and this method still remains the common choice for fine resolution studies. However, it suffers from a limited dynamic range (only 1.5-3 orders of magnitude) and low sensitivity, is laborious and gives inaccurate determinations.

Storage phosphor screen imaging systems have a much better sensitivity than film autoradiography, 250 times more sensitive than X-ray film with $^{32}$P and 60-100 times more sensitive than direct film autoradiography with $^{14}$C and $^{35}$S. The linear dynamic range of these systems are 4-5 orders of magnitude and quantification methods give results that are much more reliable than with film. However, areas having activities below the minimum threshold are not quantified accurately, and inevitably imposes a limitation to the accuracy in measurements of activity distributions.

A major practical problem of the two latter radiography modalities is the need to know the exposure time before acquiring the picture so that underexposure or overexposure may be avoided. Especially with film, where exposure times may be months, a bad estimate of the exposure time may cause waste of a considerable time.

The problems associated with the two latter radiography modalities can largely be solved by employing digital systems with direct event detection. This give results with absolute linearity, the count rate is only limited by the speed of the electronics, and the need for accurate estimates of exposure times is eliminated as one can inspect the cumulative pictures at any time. Even though the geometrical resolution of the available systems cannot compete with that of the film technique, it is comparable with phosphor screen imaging systems.

However, these techniques can not separate the contributions from different non-monoenergetic nuclides, and thus cannot be applied for simultaneous quantification of two or more different radioactive emitters at the same spot on a biological microarray. Obviously, this impedes (hinders) every application where one wants to simultaneously compare the activity from more than one specimen, such as for instance a healthy cell and a tumour cell. Thus a whole class of important diagnostic and research applications is cut off from the benefits of tagging nucleotides and/or other biological molecules with radioactive isotopes.

GB 2 311 198 discloses a method for autoradiography imaging including the steps of a) forming a subject having at least first and second markers, each marker providing radiation having characteristic energy distribution, b) detecting radiation from the marked subject using a semiconductor radiation detector having an array of cells, each cell recording a charge value dependent on the energy of incident radiation, and c) processing the output from the cells including discrimination charge values within at least two charge value ranges and allocating a display colour value to each pixel cell position in the array dependent upon the recorded charge value, and d) forming an image display with individual cell positions having a colour representative of the colour values.

Also, according to our knowledge, the known methods and apparatuses for performing the conventional radiography methods are all limited to labelling all molecules on the surface with only one radio nuclide at time and a limited number of radio isotopes. Thus there is a need for a general method for performing simultaneous determinations of the radioactivity/intensity of two or more different radio nuclides at each spot in a biological microarray. A gamma camera equipped with a parallel hole collimator may be used to simultaneously measure the distribution of several gamma-emitting radio nuclides on a surface. However, this camera cannot resolve structures with spatial extent on the sub millimeter scale.

**Objective of the invention**

The main objective of this invention is to provide a method and apparatus for performing simultaneous quantifications and/or comparisons of one or more target biological molecules that are distinctively marked with radioactive isotopes and then adsorbed onto a probe array of biological molecules deposited on a substrate, and which substantially eliminates or reduces the above mentioned problems.

An additional objective of this invention is to provide a specific method and apparatus for simultaneous determination of two or more cDNAs which are labelled with beta emitting radionuclides with distinctive distributions, and which then are adsorbed onto a DNA-microarray.

**Brief description of the invention**

The above mentioned objectives can be obtained by the features as set forth in the following description and accompanying claims.

The objects of this invention can be obtained by exploiting a digital detection system capable of a) recording the position and energy of each particle/photon that hits a detector surface, b) performing a real-time rearrangement and data reduction, and c) simultaneously estimating from the rearranged data the quantities of two or more radio nuclides present within an arbitrary region of a probe surface, e.g. by the methods recently published by two of the present inventors [5] for separating the contributions from two radioactive nuclides in an autoradiography picture.

The digital detection system or apparatus includes an electronic device for detection and imaging of small radioactive samples which is capable of registering and recording the deposited energy and position of each radioactive particle that penetrates the instrument's sensor system. This device is a further development of the instrument disclosed in US 5 656 818 and EP 0 983 705, thus, both patents are enclosed by reference. The working principle of the instrument is substantially the same as the instrument disclosed in the above cited patents. Thus we will only give a summary of the working principle of the inventive instrument here, which may be considered as consisting of two main parts:

1. A double-sided monolithic semiconductor strip sensor designed as a single plate. The plate should preferably have a larger, or at least an equal surface area than the biological microarray. Typical dimensions will be an area in the order of ten square-centimetres and a thickness of about 300 $\mu$m. The surface of the semiconductor plate is covered on one side with parallel bands or "strips" of a sensitive p-type material, and the other side is covered with parallel bands or strips of a n-type material. The n-type bands are perpendicular to the p-type bands. Thus on each intersection of the bands, a pn-diode is formed, and the bands will thus form a matrix that provides a two-dimensional reading of the hit position of the radioactive particle. That is, as long as the pn-diodes are reverse biased, they will respond with a current pulse at the two orthogonal strips they are connected to when a radioactive particle penetrates the bulk of the diode. The strip pitch is typically in the order of 50 $\mu$m. Thus for a sensitive area of 64mm x 32mm, there will be 1280x640 strips, giving the instrument a two-dimensional grid of 820 000 pixels. The semiconductor sensor plate can be made to respond to all types of radioactive particles, including $\alpha$, $\beta$, $\gamma$, and positron radiation. Depending on the energy levels and type of radioactivity, monolithic plates of Si, GaAs, CdTe, or CdZnTe etc. can be employed.

2. An electronic system for reading out the signals from the sensor. The system consist of Multi-Chip-Modules (MCM's) surrounding the sensor plate, and which comprises several parallel FE-chips (ASIC) each with an array of preamplifier and noise-filtering amplification channels. For each radioactive particle that impinges the sensor, the charge from the sensor x- and y- strip that carries the largest signal plus those of two or more neighbouring strips, will have their charge-signal integrated and filtered by the corresponding channels in the corresponding FE-chips to give the energy of the particle. In general one has that the impinging radioactive particle will deposit it's energy by ionising the bulk material of the sensor plate, thus the energy of the particle is proportional to the sum of the amplitudes of the recorded signals in the actual strips of the sensor plate. The amplified signal of the x- and the y-side is read out from the MCM's by a Data-Acquisition-System (DAQ) and the controlling computer thus receives the x-y coordinate of the impinging radioactive particle together with the energy of the event.

By employing this specific combination of solid state technology and highly integrated read-out electronics, one achieves a highly sensitive accurate and reliable instrument which records the position and energy of each radioactive particle that penetrates the sensor plate of the instrument. Thus by placing the sensor plate in close contact with the biological microarray, one can measure the radioactivity from each test spot on the biological microarray. In order to make the instrument versatile and suitable for different types of radiation, it is envisioned that the instrument is equipped with replaceable sensor plates which have equal dimension but are made of different monolithic semiconductor materials, and which can easily be inserted into the integrated read-out electronics. Thus as an example, if one employs a sensor plate made of silicon which responds well to β-radiation, and then wants to investigate a biological microarray tagged with γ-emitters, the instrument can momentarily be made ready for this by substituting the silicon sensor plate with a sensor plate made of cadmium-zinc-tellurium which responds well to γ-radiation.

During registration, the microarray glass-plate is placed face down in close contact with the semiconductor sensor plate of the instrument, separated by a 1,3 $\mu$m thick mylar foil. Thus the detector system will, due to its two-dimensional pattern of pn-diodes, register both the energy of the incoming radioactive particle and the position on the biological microarray it originated from. This implies that the summed emission-intensity must be sufficiently low to allow the instrument to register one by one emission event. The maximal activity that can be reliably measured is in the entire surface ranges from cosmic background radiation (approx. 1 Bq) up to 10MBq, but for many practical applications a limitation of 4-6 kBq is preferred.

However in the case of 6-emitters, there is a general problem with comparisons and/or simultaneous determinations of the activity since they emit at several energy levels, usually forming continuous energy spectra. That is, even if one employs two radioactive isotopes that emits β-particles with mean energies that are well distinctive, one cannot determine directly by the aid of the registered particle energy from which of the two isotopes the registered particle originated from. However, the energy spectra of the two isotopes can be measured for each radio nuclide separately, and these spectra can be employed to separate the contributions from each isotope as long as the isotopes have spectra that are sufficiently different.

In the method according to this invention, the energy spectra of the isotopes are formed by dividing the total energy span of the emitted β-particles into a vector of $k_{max}$ discrete energy bins, where $k_{max}$ is a positive integer which preferably should be larger than 20. That is, for each region, r, on the probe surface, the registered β-particles are classified according to which of the k'th energy bin they belong to. Thus a discrete vector representation of the actual

energy spectrum, $\mathrm{E}_{rk} = \sum_{ij \, \in \, r} E_{ijk}$ , of the isotopes for each region on the probe surface is achieved.

As a result of the real-time data rearrangement and possible subsequent reduction the instrument provides at the end of a pre-set measuring period, image information in the form of an image containing k energy bands (energy band images), in analogy to a colour image that contain these bands as a red, blue, green colour etc. One element of an energy band image, denoted $E_{i,j,k}$, contains the number of particles within the k'th energy interval that were recorded at digital coordinate i,j.

For any image region, r, the registered discrete energy histogram $E_{rk}$ is a linear combination of the known energy spectra from each of the actual radioactive isotopes. In the following we will illustrate the method in the case when two isotopes are applied, but one should have in mind that the method can be applied for any number of isotopes. If the known energy spectra were acquired with identical energy bins as was used for acquisition of the energy band image, and normalised so that the sum over all bins equals 1, the calculations can be performed as follows:

Let $S_{Ak}$ and $S_{Bk}$ denote the energy spectra at bin k obtained with pure samples of isotope A and B respectively. Further, let $a_r$ and $b_r$ be the unknown contributions within region r (total number of recorded emissions) from isotope A and B, respectively. It is important that the energy bins of the reference spectra are identical to those used during the data acquisition of the actual measurement leading to the energy band images ($E_{rk}$).

Then:

$$E_{rk} = s_{Ak} a_r + s_{Bk} b_r \qquad\qquad (1)$$

These relationships can be employed in one of the above mentioned algorithms from the article by [5] to estimate

contribution $a_r$ and $b_r$ for each region on the probe surface. The article gives two general algorithms, the least square method (LS) and maximum likelihood method (ML). Both are incorporated here as a reference, and all we need to say is that the LS-algorithm is the simplest and requires considerable less computing power than the ML-algorithm, but suffers from a slightly larger inaccuracy in the estimates. Thus we will focus on the LS-algorithm in the following discussion, but nevertheless emphasise that both algorithms are incorporated in the invention.

In principle the LS-algorithm works as follows: The aim is to find which contributions $a_r$ and $b_r$ gives the least error sum $S_r$ over the whole vector, where $S_r$ is given as:

$$S_r = \sum_{i=k}^{k_{max}} (E_{rk} - s_{Ak} a_r - s_{Bk} b_r)^2 \qquad (2)$$

The minima is determined by:

$$\frac{\partial S_r}{\partial a_r} = 0 \qquad \text{and} \qquad \frac{\partial S_r}{\partial b_r} = 0 \qquad (3)$$

As mentioned, the method is given as a determination of the contribution from two isotopes. The method can however be employed for any number of isotopes by simply adding a term of type "$s_{Ck}{}^*{}_{Cr}$" for each additional isotope in the equations.

In practice, the method is employed as follows: For each pixel ij of the sensor plate, there a vector $E_{ijk}$ with $k_{max}$ counters is established in the memory system of the instrument. The entire data set, $E_{ijk}$, is initially set equal to zero. When the x'th, y'th pixel registers a β-particle, the x'th, y'th counter with appropriate bin number k is increased by one. This process continues until a sufficient number of events are registered to give a statistical representation of the energy spectrum, which then is applied in one of the algorithms to determine the contribution from isotope A and B in the registered energy histogram at each region r of the probe surface.

In order to obtain a flexible instrument for a wide range of applications, the instrument is equipped with a very high resolution sensor plate containing a large number of pixels. Thus, for many applications the resolution of the biological microarray will be coarser than the resolution of the sensor plate. In these cases, considerable computing time can be saved if the registered data from neighbouring pixels can be condensed by adding them to the centre pixel. That is, by adding e.g. the registered events in one pixel and all of its nearest surrounding neighbours (a total of 9 pixels) into the centre pixel, the resolution and thus number of computations are decreased by a factor 9. If even coarser resolution is wanted one can include all neighbour pixels in two surrounding rows (a total of 25 pixels) and so forth. In this way we simulate a coarser sensor resolution and thus reduces the necessary computing steps accordingly. The spatial condensing into regions does also give an important increase in number of registered events for each element $E_{rk}$, in the resulting condensed energy band image, thus leading to an improved statistical accuracy of the estimated contributions.

Also, the method requires in advanced knowledge of the expected energy spectrum of each actual radionuclide. This can be obtained by calibration of the instrument with surface distributed sources of each of the actual radionuclides before determination of the unknown sample.

The calibration procedure is incorporated as an automatic procedure in the instrument, where measured spectra are obtained for each of one side (the x-side) strips of the sensor plate. By determining the first and second moment of these spectra, their zero offset and gain with reference to a reference strip can be determined (note that for the calibration procedure, neighbour strip deposited energies are not taken into account). These calibration data can then be used in real time to correct the energy of each detected radioactive event. Also, this calibration procedure avoids problems encountered with external photon (gamma) sources where scattered radiation gives rise to changes of the spectra.

Since the glass plate containing the biological microarray may be slightly rotated with respect to the principal axes of the instrument, the rotation angle must be determined so that the test spots on the microarray form exactly vertical and horizontal rows and columns in the image. The determination of the optimal angle may be carried out as follows: A sum image is formed by adding all bands in the energy band images, $E_{ijk}$, i.e. summation over the index k. This image is then rotated in steps of 0.1 degrees, and for each new angle, the projection of the image on the x- and y-axes, respectively, are calculated. The optimal rotation angle corresponds to the maximum of the variance (i.e. the maximal contrast variation). The projections can easily be used to determine the upper, lower, leftmost, and rightmost position of the matrix. Thus, identification of a test spot is easy, and the associated region occupied by that test spot can be identified. It is thus possible to create a new energy band image where each point corresponds to the region occupied by one test spot. This will significantly strengthen the statistical accuracy of the final results. The determination of the outer boundaries of the matrix may also be determined by correlating the matrix to a virtual matrix. This procedure has the benefit of not requiring an input from the user.

This method and apparatus have been described as a general approach suitable for simultaneous determinations of the contribution from more than one radioactive isotope of a registered energy spectrum, which is a superposition of unknown quantities from each isotope. The method is obviously necessary in the case of β-emitters since these have overlapping energy spectra, but may seem unnecessary for other types of radiation which has a more monoenergetic nature and thus gives very distinct energy spectra which in general do not overlap each other. But the method according to this invention may be beneficial even for these cases since the method allows to employ radioactive isotopes which emit photons or particles with small energy differences because the method will separate possible overlapping energy contributions. Also, the method will automatically treat the problem with secondary registrations due to background radiation and unavoidable scattering, and thus give determinations with excellent precision. This feature is an obvious benefit for all types of radiation.

**Brief description of the drawings**

Fig. 1a is a schematic view of the sensor plate with strips, and a schematic representation of the two-dimensional array of pn-junctions (diodes).

Fig. 1b is a graphic representation showing a typical distribution of the measured deposited energy in the strips in the vicinity of the hit position on the sensor plate. In the figure, the radioactive particle hits the sensor plate at the point marked with a cross and terminates at the point indicated by the arrow.

Fig. 1c is a flowchart schematically illustrating one amplifier channel.

Fig. 1d is a schematic representation of the sensor plate and the surrounding electronics for determining the hit position and energy of a radioactive particle.

Fig. 2 is a flowchart schematically illustrating a preferred embodiment of the invention in the case of simultaneous determination of two or more cDNAs which are labelled with beta emitting radionuclides with distinctive distributions, and which then are adsorbed onto a DNA-microarray.

Fig. 3 is a block diagram schematically illustrating the data processing for separating the contribution from each radio isotope.

Fig. 4 is a chart showing the energy spectra for $^{33}P$ and $^{35}S$ isotopes as measured by the apparatus according to the invention, using the actual numbers of energy intervals (energy bins).

Fig. 5 shows the x-axis projection of the determination of salmon sperm labelled with $^{33}P$ and $^{35}S$, both separately and in combination.

Fig. 6 shows a virtual fluorophore plot of a simultaneous determination of $^{33}P$ and $^{35}S$ labelled RNA-lines from OHS osteosarcoma and MCF-7 breast cancer cells, respectively.

**Detailed description of the invention**

The invention will now be described in detail with reference to the enclosed drawings and an example of a preferred embodiment of the invention.

As mentioned, the device for registering the hit position and energy of incoming radioactive particles comprises two parts: a double sided strip sensor plate with orthogonal strips, and a surrounding readout electronics which measures the captured signal generated by the sensor plate upon the impact of the radioactive particle.

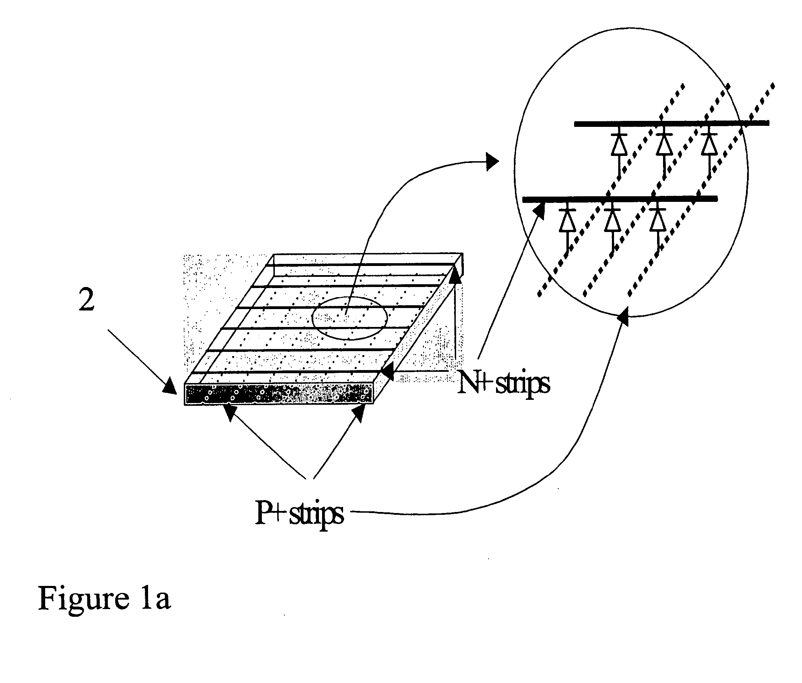

Figure 1a gives a schematic representation of the sensor plate 2 which shows the arrangement of the orthogonal strips on both sides of the sensor plate, and the forming pn-junctions (diodes) inside the sensor plate at the crossing points of the strips.

**EP 1 358 357 B1**

The sensor plate is in general a well known device mostly utilized for physics research detectors. In the case of registering beta-emissions, it is a rectangular piece cut from a processed high-resistivity silicon wafer. It is typically 300 micrometer thick and the size is typically some ten square-centimeters. A practical, more specific example is a 6.4cm x 3.2cm device. There is implanted narrow-pitched low-resistivity strips across the wafer on both sides. The strips on one side are made orthogonal to the strips on the other side, and the implant on one side is P+ whereas the implant on the other side is N+. The strips are typically spaced with 50 micrometers which, for the size-example above, would give 1280 strips on one side and 640 strips (orthogonally) on the other side. Because of the P+/N+ implant of the strips, each strip crossing will become a P+/N+ (pn-) junction meaning that there will be a small diode at each crossing. In turn, this means that the device in this example will consist of a two-dimensional array of 1280 x 640 pn-junctions (diodes). This provides the device with position resolution (imaging) properties. For the diodes to become active, a bias voltage must be applied between the strips on one side and the strips on the other side. The bias must be negative on the P+ side in order to reverse-bias the diodes. Now the diodes are biased in the reverse direction, and only a tiny leakage-current will flowing.

As a radioactive particle emits from the sample-martrix and enters the sensor plate, it will penetrate into the bulk of the sensor (between the orthogonal strips) and give it's energy away inside the crystal as it travels. In most cases related to the practical usage of the sensor, it will give away all it's energy and finally stop. The given away energy will ionize the bulk which contains an array of reverse-biased diodes. Depending on the angle of penetration, one or more of the diodes may contain the bulk affected and ionized by the moving particle. Every diode affected will respond to the ionization by the release of a charge which is proportional to the energy given away by the particle to this diode. Thus, by summing the charge released by all the affected diodes, it is possible to tell what the energy originally was in the penetrating particle. And consequently, double sided detectors becomes a detection device allows measuring both the position of individual impinging particles and their energy.

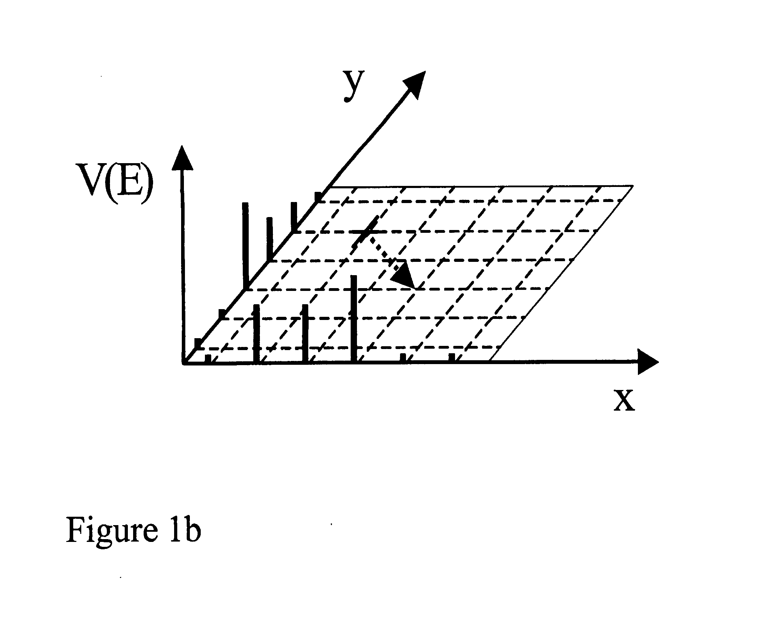

Most particles will penetrate the sensor in a certain angle, i.e. not perpendicular to the plane (se Fig. 1b). The common case is then that the particle has sufficient energy to travel a certain distance, at a certain angle, and consequently make a path through the bulk of several diodes. The crossed strip structure does not allow to determine exactly which diodes this is, because reading the strips only provides an x- and y- projection of these diodes. I.e., one can identify a rectangle containing the diodes affected. Such identification is important in this invention, because it can help identify the point where the particle entered the sensor and thus enhance the "imaging" quality.

As mentioned, it is of importance to measure also the energy of the signal. The total energy is found by summing the signal from the strips. However, the energy profile of the strips can also be of importance, since this is needed for the final identification of the entrance point of the particle. Due to the physical properties, a particle moving in the bulk, will ionize the material most as it is stopping. I.e. the strips where the highest energy is measured will be the projected position of the stopping point, and consequently, the entrance point can be determined by this knowledge.

The silicon sensor is useless without having the means to read out the strip charge signals to a computer. This is performed by the surrounding readout electronics which is invented to perform the readout of the particular properties of the sensor described in the section above. A minimum configuration of electronics for the sensor to be useful is that each strip has an amplifier attached in order to transport the charge generated in the sensor into a measurable voltage on the output of the amplifies. The voltage change on the output of an amplifier will be proportional to the amount of charge released by the diode(s) attached to the strip, and hence also proportional to the energy given away by the particle in the region belonging to this strip. In Figure 1b, it is illustrated how the energy disposition of a particle is measured by the amplifiers. The particle has entered the sensor plate at the point indicated by a cross. Then it moves downwards, not indicated, but typically also with a horizontal movement component as shown by the arrow. The strips affected will cause a voltage amplitude (V) at its attached amplifier outputs representing the energy (E) of the energy deposited to the actual strips. The strip signal amplitudes of the example are indicated by the vertical bars. The sum of the bars on one side represent the total amount of particle energy given away, and the highest bars can, as mentioned, be used to identify the endpoint of the particle movement.

It should be emphasized that the example shows very few strips. In reality, the number of strips will be large, but the typical number of strips affected will be close to the number used in the example. This means that this method can be used to marginally increase the spatial resolution, but relatively seen, not near to how it appears in the example. In many applications, determining just one of the strips affected would be sufficiently acceptable.

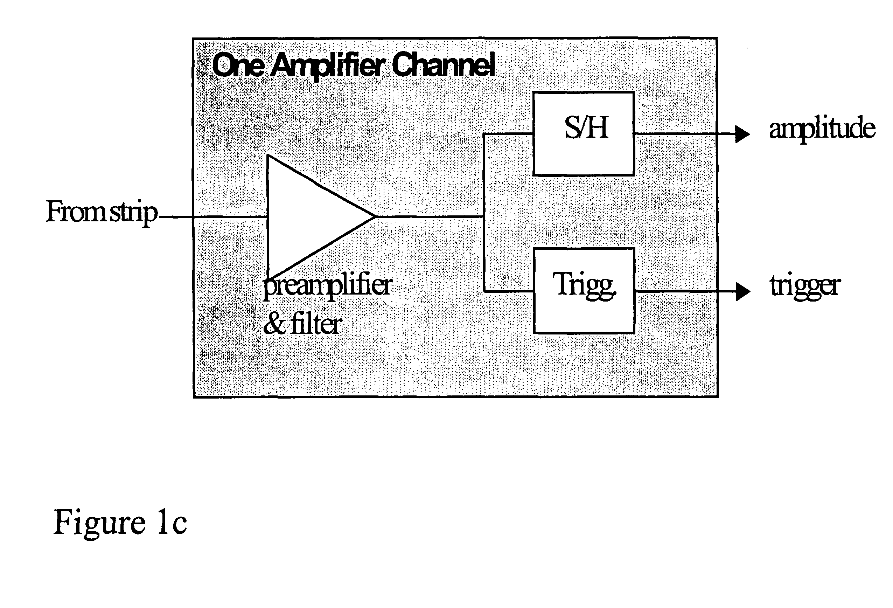

In order for the electronics to be usable in order to capture the strip signals by a computer, each amplifier must be followed by a triggering unit in parallel to an amplitude measuring unit. This is illustrated in Figure 1c. The first unit, that includes a discriminator with a predetermined threshold, will tell the system that a particle has been detected. The other unit, which includes a sample/hold, will store the energy-dependent amplitude value of the strip.

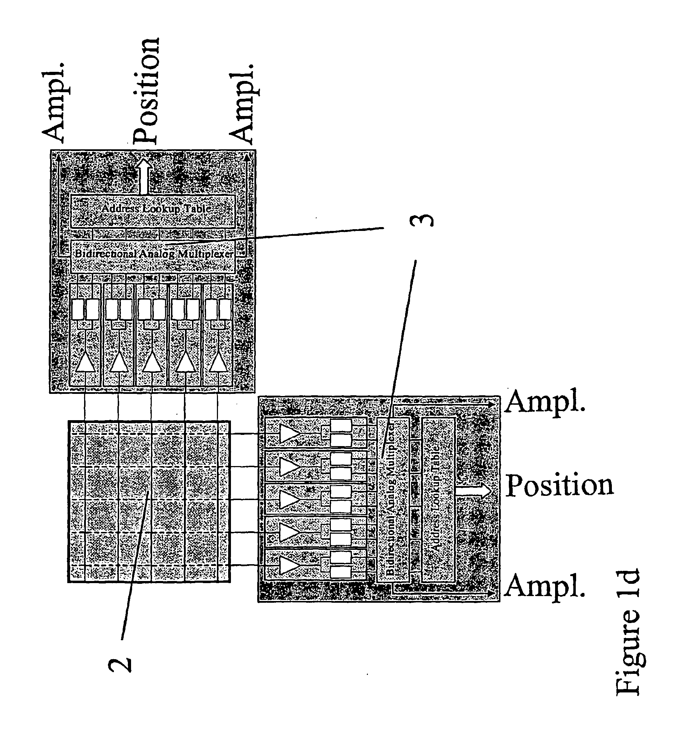

Figure 1d shows the full architecture of surrounding electronics that handles the transfer of the proper data, originally generated by the particle, into the system. Both sides of the sensor will have the same type of electronics which typically is integrated in an ASIC. An ASIC only have space for a given number of channels and therefore, several ASICs may be placed next to each other to cover one full side of the sensor (all the strips). The electronics shown behind

8

the channels will be on the same chip as these, but made such that when more than one chip is used in parallel, it will operate as if it was all on one single chip. Consequently, the example can be interpreted as if all the electronics on one side is integrated in one ASIC.

The function of such ASIC is the following: All the channels will in parallel sense the signals on the strips. As a particle is impinging the sensor, one of the channels will detect this by its triggering unit. More than one channel can actually detect the same signal, but internal arbitration will make sure that only one will. Upon such detection, a trigger signal will be sent to the external system (not described herein), instructing it to read the data off the chip. Upon such action, the chip will provide the system with the position (address) of the channel that generated the trigger. It will also sequentially provide the system with signal amplitudes of a number of strips near the center of interaction. The analog values will be transferred in a sequential stream, starting with the amplitude of the same channel that generated the trigger, then continue with the amplitude of the next strip in one of the directions and so on. The strips in the opposite direction will be read in the same manner simultaneously to the readout of the other direction, and starting from the same point. This concept provides fast readout and flexibility. The flexibility arises from the fact that it is not necessary to scan all the strips, but only those near to the triggering strip. As mentioned before, this will typically only be some few. The system can chose how many strips it wants to read out.

A system clock will be synchronizing the data transfer. The position address may or may not stay on the position data-line throughout the streaming of the amplitude values.

The other side of the sensor will have the identical structure and function in the exact same manner as of the side just described. Both sides readout will be necessary in order to gather the desired position information.

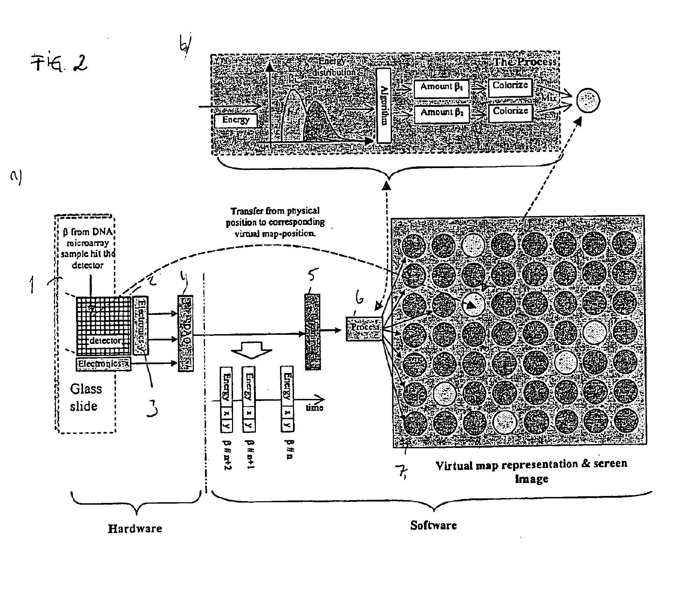

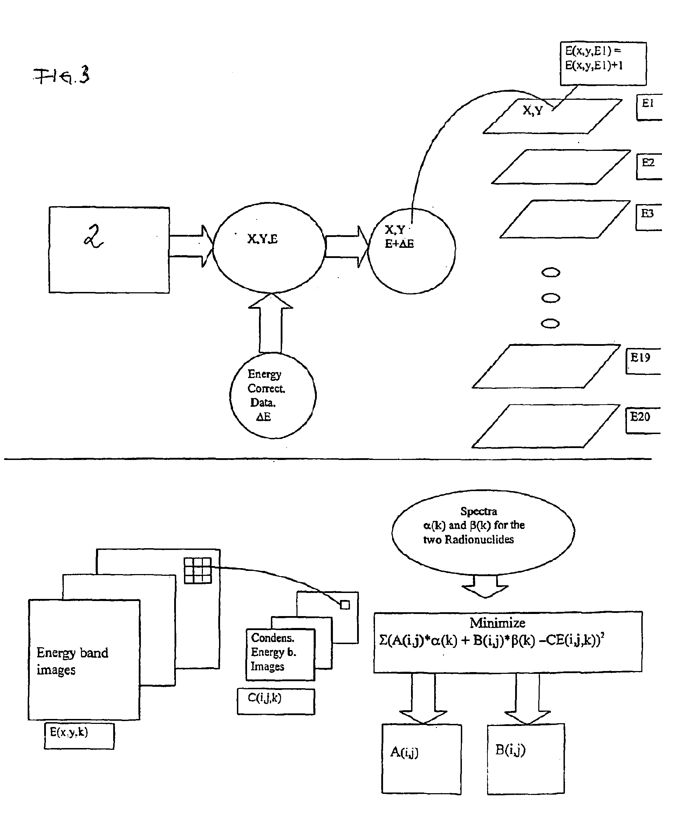

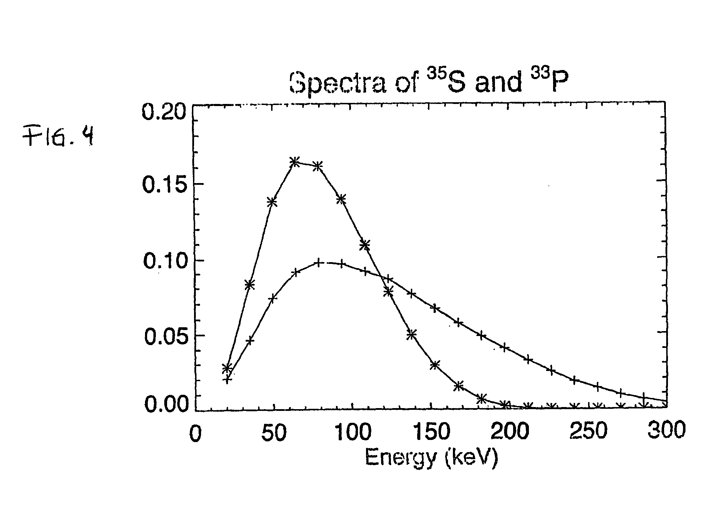

By connecting the above described sensor plate and readout electronics to a data acquisition unit and a computer unit loaded with a software that is able to execute one or both of the algorithms given in [5], a preferred embodiment of the inventive method for determining the contribution from each radioactive isotope on the microarray can be schematically presented as in Figs. 2 and 3. The example is given in the case of determining the activity of DNA-molecules that are marked with two distinctive β-emitters and adsorbed onto a DNA-microarray. From the discussion above, the energy spectrum of the two radionuclides should be essentially distinct and have appropriate decay properties and energy levels, allowing determining beta emissions within a suitable time range. Two suitable radionuclides for this purpose are $^{35}P$ and $^{33}S$. The energy spectra of pure samples of these isotopes are given in Figure 4.

In Figure 2, a glass slide containing a DNA-microarray labelled with unknown quantities of the radioactive 2-emitters $^{35}P$ and $^{33}S$ are marked with numeral 1. The glass slide is placed beneath and pressed towards a quadratic monolithic semiconductor sensor plate 2 made of silicon in such a manner that the DNA-microarray is covered by the sensor plate. The sensor plate 2 and the DNA-microarray are separated by a 1,3 μm thick mylar foil (not shown). Thus, when a β particle is emitted from one of the radionuclides, it will hit the sensor plate 2 and create a small current pulse in a pn-diode which lies substantially directly above the radionuclide that emitted the β-particle. The readout electronic system 3 of the apparatus will pick up the position (x,y-coordinate) of the pixel and thus the actual test spot on the DNA-microarray from which the β-particle originated. The electronic system will also measure the energy of the β-particle and send this information in digital form to a data acquisition unit 4 which accumulates all recorded events and organises them according to the registered position and energy. The data acquisition unit 4 may comprise any conventional digital storage unit such as RAM-memory chips, hard-disc, floppy-disc, CD-ROM etc.

When the data acquisition unit has received sufficient data for making a statistically reliable analysis, i.e. when the elements of $E_{ijk}$ carry sufficient information, the registration of events from the sensor plate is stopped.

Then the recorded energy band image constitutes the input for the software program that determines the amounts of each isotope for every pixel, or after a condensation into regions as described earlier, at every test spot on the microarray by employing one of the algorithms given in [5]. That is, the algorithm determines how much (with how many registered events) each radionuclide did contribute to $Eij_k$, or $E_{rk}$ in the case of a larger spot (A proper quantification will of course require knowledge of the nucleotides half-time and the detector sensitivity for the actual radionuclide (registered events per unit of activity on the surface)). This procedure is schematically given in the highlighted box in Figure 2 and in Figure 3. The figures depict a case with two β-emitting radionuclides that are being determined by the least-square algorithm and where the pixels are condensed by a factor of 9 (all surrounding pixels in one layer are added into the centre pixel).

One advantage with this method is that the amounts of each isotope at every test spot are determined in absolute quantities. This gives several options for presenting the information. One option in the case of two isotopes is to determine the ratio of the isotopes at each spot and present this in a virtual map representing the biological microarray. Another option can mimic in a single image the colours obtained with red/green fluorophore techniques: By assigning the intensity of the image of one of the radionuclides in a red colour and the other in a green colour, a colour scale that mixes red and green in different proportions is created. It is also possible to present histogram-charts which show the absolute quantities of each radionuclide.

Determination of absolute quantities of RNA

The feasibility of the preferred embodiment of the method and apparatus will now be demonstrated in the case of determinations of absolute quantities of RNA marked with known quantities of $^{33}P$ and $^{35}S$.

After calibration of the instrument, and prior to the measurements of the DNA microarray, the spectra of $^{33}P$ and $^{35}S$ were measured separately as follows: Solutions of the radionuclides were deposited evenly on glass slides and allowed to dry. The multiple energy band images obtained with these slides had an energy bin size of 15 keV, and there were 20 bins starting at a lower threshold of 20 keV. The spectra were created using the same bin sizes and number of bins and assumed to be independent of the position on the detector, therefore the entire images contributed to the spectra. For each pixel (or dot), the unknown contributions P and S from $^{33}P$ and $^{35}S$ respectively, were determined by the regression analysis and also by the Maximum Likelihood procedure. The resulting images P and S were displayed as one single image in the mode commonly used for presentation of images obtained with fluorophores. The phosphorus image (P) was sent to the red channel of the display, and the sulphur image was sent to the green channel. Equal amounts of red and green thus gave a yellow colour.

The DNA-arrays of all examples were produced adhering to the following protocol:

*Coating:*

Poly-L-lysine coated, pre-cleaned microscope slides from J. Melvin Freed Brand (Sigma.Cat.No.S-8902) with dimension 75x25 mm were employed for printing. The slides were lysine-coated by placing them in a NaOH/EtOH-solution for 2 hours on an orbital shaker (75 g NaOH, 300 ml $ddH_2O$, and 450 ml 96% EtOH), then transferred to high purity water (double distilled, $ddH_2O$) and plunged up and down at least 10 times until the slide surface became clear. The slides were then repeatedly rinsed in fresh $ddH_2O$ with shaking, soaked in poly-L-lysine solution (40 ml poly-L-lysine, 40 ml PBS and 300 ml $ddH_2O$) on an orbital shaker for 1 hour. Finally, the slides were repeatedly rinsed in $ddH_2O$ and then spun dry (500rcf for 5 minutes) followed by exposure to 55°C for 1 hour. The slides were allowed to age for at least two weeks at room temperature before printing.

*Printing:*

A home made robot was employed as an array printer, made by a description from P. Meltzer at NIH. Carbide split pins produced by Beecher Inc. were used, printing 48 slides with 8 pins (2x4) in one run. The pins were rinsed by soaking them in 1 M NaOH for 2 minutes, followed by 10% SDS for 2 minutes and a short wash in $ddH_2O$. Then they were given a short ultrasonic treatment for 10 seconds before an additional rinsing in $ddH_2O$. The pins were then blown dry with pressurized air. The DNA was printed directly from Costar 96-wells plates (U-bottom, polystyrene cat.no.3367), where DNA PCR products were precipitated and dissolved in 25 $\mu l$ 3xSSC. The printing were performed with a humidity of about 50%. The temperature was about 27-30°C during the printing session. It were employed arrays containing 2200-2700 genes.

*Post-processing:*

The slides were treated with a UV-dose of 100 mJ to cross-link DNA to poly-L-lysine. A blocking solution was made by dissolving 6.0 g of succinic anhydride in 335 ml 1-methyl-2-pyrrolidinone and 15 ml of 1 M Boric acid (pH 8.0) under stirring. The slides were then submerged and plunged into the solution and put on an orbital shaker for 15-20 minutes. The slides were given a short rinse in $ddH_2O$, then submerged in hot water (95°C) for 2 minutes and then transferred to cold 96% EtOH for 30 seconds. Finally the slides were dried (500 rcf. for 5 minutes) and stored, ready for use.

The preparation of radioactive cDNA probes for all experiments were performed according to the following protocol:

*Conversion of 2-5 $\mu g$ of total RNA into $^{33}P/^{35}S$-labelled first strand cDNA:*

An a-33P-dATP, 10 mCi/ml, cat. no. AH9904, Amersham Pharmacia Biotech and an a-35S-dATP, 10 mCi/ml, cat. no. AG1000, Amersham Pharmacia Biotech were used for incorporation of radioactivity.

RT-reaction was made on ice:

| | |
|---|---|
| 5 x First strand reaction buffer | 4.0 $\mu l$ |
| RNasin | 0.5 $\mu l$ |
| DTT 100 mM | 2.0 $\mu l$ |

(continued)

| | |
|---|---|
| Anchored Oligo-dT 2μg/μl | 1.0 μl |
| 20 x low-dA/NTP mix | 1.0 μl |
| a-33P dATP/a-35 dATP | 4.0 μl |
| 5 μg total RNA+$H_2O$ | 6.5 μl |
| | 19.0 μl |

The reaction was incubated at 65°C for 5 min. and then cooled to 42°C for 5 minutes. 1 μl SuperScript II enzyme was added and further incubation at 42°C for 30 minutes was performed. Another 1 μl SuperScript II enzyme was added and incubated further at 42°C for 30 minutes. The reaction was stopped by adding 5 μl 500 mM EDTA, and 10 μl 1 M NaOH was added and the mixture was incubated at 65°C for 60 minutes to hydrolyse the RNA. The mixture was then cooled to room temperature and 25 μl 1 M Tris-HCL (pH 7.5) was added to neutralise the solution. Finished probes were stored on ice until cleanup.

*Probe cleanup and preparation:*

The probes were cleaned by the use of Microcon columns. 300 μl 1 x TE was added to each RT-reaction and the two differently labelled samples were combined before transferring to the Microcon column and spun at 13 K until 30-50 μl solution was left. Following repeated additions and spins, a final cleaned volume of 10 μl was reached. The column was inverted into a new tube and spun in one minute at 13 K to recover the probe. In a final probe volume of 18 μl (for a cover slip size of 22x20 mm), the following was included: 1 μl 10 μg/μl COT-1 DNA, 1 μl 8 μg/μl poly A, 1 μl 4 μg/μl yeast tRNA, 1 μl 10 μg/μl BSA, 1 μl 50 x Denhardt's solution, 3.1 μl 20 x SSC (final concentration 3.5 x SSC), and 0.5 μl 10% SDS (final concentration 0.3%). The probe was then heated in boiling water for 2 minutes before spinning at 13 K for 10 minutes.

Example 1

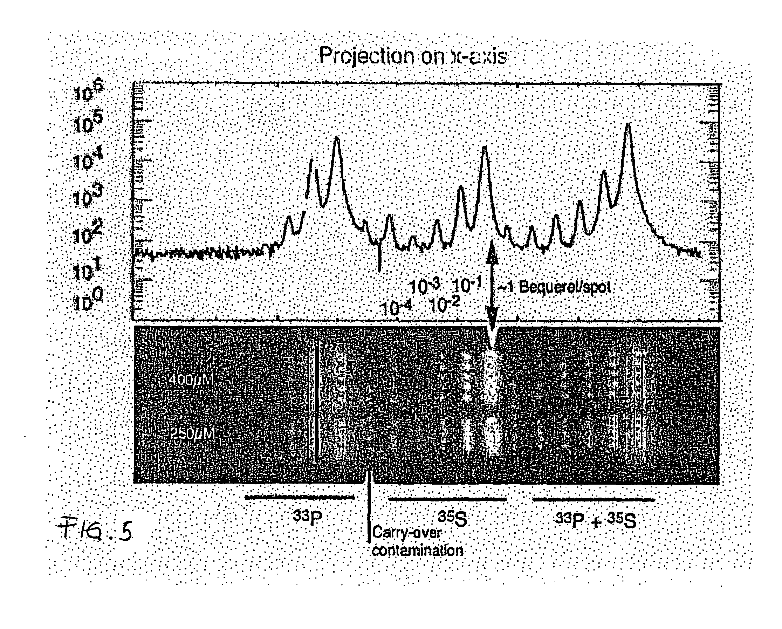

To demonstrate the feasibility of the method and apparatus according to the invention, a model experiment with the two isotopes $^{33}$P and $^{35}$S diluted in salmon sperm DNA and spotted onto a glass slide to give an approximate radiation of 1 Becquerel per spot undiluted were performed. In addition a dilute series was made to dilute the 5 logs in order to demonstrate the dynamic range of the method and apparatus according to the invention. This was performed separately for each of the two isotopes, and for a combination of the isotopes. Two spot grids were employed with a centre distance of 250 μm and 400 μm. The result for all three experiments are given in Figure 5, both as a fluorophore plot and in the form of the recorded energy spectrum as a projection of the signal onto the x-axis in order to show the quantitative relationship with dilution.

Example 2

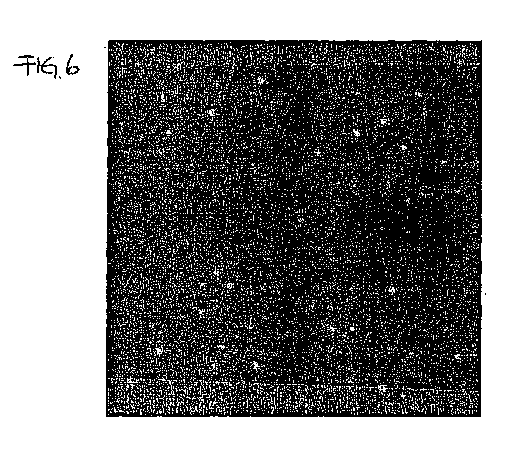

In order to verify the applicability of the method and apparatus according to the invention for microarray applications, an experiment using two cell line RNAs in reduced amounts were performed. Otherwise standard microarray procedures were employed. By using RNA from two different cell lines, OHS osteosarcoma cell line and MCF-7 breast cancer cell line, a demonstration of a successful separation of the nuclides are performed, and given as a fluorophore plot in form of an overlay pseudo-colour image of the two isotopes in Figure 6. The figure demonstrates clearly that the method and apparatus according to the invention achieves the dynamic variation of signals that may be expected from similar experiments using fluorophores, but with significantly lower starting RNA-material.

As mentioned, radioactively labelled biological substances are easily incorporated into living cells as opposed to most fluorescent substances. This may be done by adding medium substituents, such as nucleotides, amino acids, or other components where radioactive isotopes have previously been incorporated. This possibility allows for a range of new applications, in which two are described here:

Example 3

The amount of any protein in a cell is partly regulated by the stability of it's corresponding RNA. This is generally considered to be related to the size of the poly-A tail, and partly other sequence-specific factors. To date, examination of individual mRNA species have been the method of choice. By the incorporation of two radioactive labels, it becomes possible to monitor all mRNA species in a population by comparison with a standard. This permits a genome wide scanning of RNA half life, when combined with DNA microarrays.

Instead of using cDNA with radioactivity in a cell free system enzymatically, a35S-UTP and a33P-UTP are added to the culture medium of parallel cell cultures in appropriate concentrations at time 0. Then the cells are allowed to grow until time 1, when the radioactive nucleotides are sufficiently incorporated to allow detection of RNA produced in the cells from time 0. At time 1, one parallel is treated chemically to block RNA synthesis. At time 2, both cultures are harvested, RNA is isolated, and equal amounts of RNA from both cultures are mixed. This mixture is then treated as the combined probe of cDNA in example 1 and hybridised to the microarray.

Finally the signals are recorded and analysed to find the contribution from each nucleotide in order to find the ratios. This data reflects the relative half-life of all RNA species that are detectable in the cell line. This novel procedure allows for genome wide monitoring of the important cellular regulatory function of RNA half-life in conjunction with the traditional information obtained from microarray analysis in the standard format.

Example 4

In the emerging field of proteomics, similar methods to the one given in example 3 may be applied when using amino acid tags. For instance, the monitoring of various phosphorylation degrees of proteins, a major regulatory phenomenon of all cells, may be performed by incorporating radioactivity labelled tags that are added to the medium. By printing antibody arrays, the incorporation of phosphor tags in a number of different proteins may be monitored simultaneously.

Although the invention has been described as a case of examples of comparison tests of two DNA/RNA-substances tagged with a distinctive β-emitting radionuclide each, it should be emphasised that the method and apparatus according to the invention is a general approach for determining the amounts of radioactivity tagged biological molecules that are adsorbed onto a biological microarray. This applies for all types of radioactivity including α, β, γ, and positron radiation and all radioactive nuclides, especially those that may be incorporated into the target molecules such that the radioactivity is tuned to the order of 1-100 Becquerel from each spot of the microarray. Also, even though the method is especially suited for the case of radioactivity with overlapping energy spectra (β-emitters), one should be aware of that the statistic treatment of the registered radioactive events in the method gives advantages for monoenergetic radiation as well, especially when one applies radionuclides which emit radiation with small energy differences. These advantages include excellent filtration/separation of background noise (secondary radiation etc.) and a very high sensitivity. The method and apparatus is also designed for being applied on any number of different radioactive nuclides ranging from 1 and up. There are no theoretical limit to this number, but a practical limit is less than approx. 20, preferably less than approx. 10, and even more preferably less than 5 different radioactive nuclides.

**References**

1. Wang, E., Miller, L.D., Ohnmacht, G.A., and Liu, E.T. (2000), "High-fidelity mRNA amplification for gene profiling", Marincola FM Nat Biotechnol, 18(4), 457-459.

2. Nilsen, T. W., Grayzel, J. and Prensky, W. (1997),"Dendritic Nucleic Acid Structures", J. theor. Biol., 187, 273-284.

3. Wang, J., Rivas, G., Fernandes, J. R., Jiang, M., Paz, J. L. L., Waymire, R., Nilsen, T. W., and Getts, R. C. (1998), "Adsorbtion and Detection of DNA Dendrimers at Carbon Electrodes", Electroanalysis, 10, 553-556.

4. Wang, J., Jiang, M., Nilsen, T. W., and Getts, R. C. (1998), "Dendritic Nucleic Acid Probes for DNA Biosensors.", J. Am. Chem. Soc., 120, 8281-8282.

5. Kvinnsland, Y. and Skretting, A. (2000), "Methods for separation of contributions from two radionuclides in autoradiography with a silicon strip detector", Phys.Med.Biol., 45, 1183-1193.

**Claims**

1. Method for simultaneous quantification of the amounts of one or more radioactive nuclides within arbitrary regions on a probe surface where these nuclides have been deposited, adsorbed or fixed, where each of the radioactive nuclides serve as markers on compounds that have been incorporated into target tissue sections, target chemical compounds, or biological target molecules that have been deposited, adsorbed or fixed to chemical or biological probe substances on the probe surface,
**characterised in**

- **that** both the position and energy of the emitted particles and/or photons from each region of the probe surface are registered, and
- **that** the registered information from each region is employed to determine, in absolute quantities, amounts and/or activities of each radionuclide present in that region, by any suitable statistical evaluation of the information.

2.  Method according to claim 1,
    **characterised in**

    - **that** both the registered position and energy of the emitted particles and/or photons from each region of the probe surface are stored and accumulated as an energy band image, that is, stored as a vector of $k_{max}$ energy bins for each region r over the whole probe surface.

3.  Method according to claim 2,
    **characterised in that**

    - the statistical evaluation includes the least squares method, whereby a linear combination of the pre-recorded energy spectra for every radioactive marker - the unknown coefficients being the contributions from each of the radionuclides-are fitted to the data from an image element or a region of an energy band image selected from a measured composite energy histogram or a vector, and/or
    - the maximum likelihood method, whereby the measured energy spectrum for every radioactive marker is employed to find the most probable number of hits from each radioactive marker in each energy bin which would give the registered energy histogram or vector, thereby providing the total number of events originating from each of the radionuclides.

4.  Method according to claim 2 or 3 wherein the method is performed on biological microarrays where the probe regions have diameters of less than 200μm, or on macroarrays where the probe regions have diameters of more than 300μm.

5.  Method according to claim 1 to 3, **characterised in that** the one or more radioactive nuclides emit either alpha, beta, gamma, or positron particles and/or photons, and that they are incorporated into target molecules such that the radioactivity is tuned to the order of 1-100 Bq from each probe region on the probe surface.

6.  Method according to claim 5, **characterised in that** the radioactive nuclides are non-monoenergetic radioactive labels, including beta-emitting nuclides, which give partial and/or fully overlapping energy spectra.

7.  Method according to claim 6, **characterised in that** the beta-emitting radionuclides includes $^{33}$P and [35]S.

8.  Method according to claim 7 wherein the method is for monitoring the entire mRNA population in a sample from a cell type/line by comparison with a sample from a standard cell type/line, where

    - a35S-UTP and a33P-UTP are added to the culture medium of the parallel cell cultures in appropriate concentrations at time 0,
    - then the cells are allowed to grow until time 1, when the radioactive nucleotides are sufficiently incorporated to allow detection of RNA produced in the cells from time 0,
    - at time 1, one parallel is treated chemically to block RNA synthesis, and
    - at time 2, both cultures are harvested, RNA is isolated and equal amounts of RNA from both cultures are mixed, and finally
    - the mixture is treated as a combined probe of cDNA and hybridised to a biological microarray, from which the total quantities of both radioactive markers can be determined.

9.  Method according to any of claims 1 to 7 wherein the method is for simultaneous monitoring of various sets of protein degrees of phosphorylations by incorporating radiolabelled amino acid tags into the medium and then adsorbing them onto biological microarrays containing structured sets of antibodies.

10. Apparatus for performing simultaneous quantification of the amounts of one or more radioactive nuclides within arbitrary test regions on a probe surface where these nuclides have been deposited, adsorbed or fixed, **characterised in that** the apparatus comprises

- an electronic system for real-time measurements of hit positions and deposited energies of radioactive particles/ photons that are emitted from the test regions of the probe surface, and which comprises a multi-strip semiconductor detector with orthogonal p-type and n-type strips on opposite sides, respectively, and readout electronics that are capable of detecting the hit position, defined by x,y-coordinate, on the detector for each radioactive event as well as the deposited energy of the particle/photon, a loading mechanism that allows the probe surface to be positioned in contact with the semiconductor detector over its entire surface, separated only by a thin foil of mylar for protecting the semiconductor detector,
- a real time data organisation module which keep track and stores the position and energy of every registered radioactive event, and which divides the registered events for each pixel, defined by x,y-position, of the semiconductor detector into a vector of k energy bins, thus forming an energy band image,
- a computer module comprising hardware and software which is capable of employing the image band image to determine, in absolute quantities, the amounts or activity of each radioactive label present on any pixel or condensed region of the semiconductor sensor plate, and
- a module for displaying the distribution of the activity of each radioactive marker across each pixel of the semiconductor plate, or optionally, in selected test regions on the probe surface.

**11.** Apparatus according to claim 10, **characterised in that** the readout electronics for real-time measurements of hit positions and deposited energies of radioactive particles/photons comprises

- one amplifier channel consisting of a preamplifier and filter unit, and a triggering unit and sample/hold unit in parallel for each strip, and
- a data-acquisition-system in communication with the amplifier channels and which is capable of keeping track of the position, defined by x,y, and energy of all registered radioactive events that hit the sensitive part of the semiconducting sensor plate.

**12.** Apparatus according to claim 10 or 11, **characterised in that** the semiconductor detector comprises a quadratic plate of a monolithic semiconducting material with dimensions of ten square-centimetres and a thickness of approximately 300 $\mu$m and which has a sensitive area of 64 x 32 mm$^2$, and where the strip pitch of the sensitive n-type bands and the orthogonal oriented p-type strips on the opposite side of the semiconductor plate is 50 $\mu$m, giving a total of 1280 x 640 strips or a two-dimensional grid of 820000 pixels.

**13.** Apparatus according to claim 10 to 12, **characterised in that** the loading mechanism and semiconductor detector are designed such that the semiconductor plate can be taken out and replaced with another semiconductor plate with similar pixel-design and dimensions, but of another semiconducting material in order to allow the apparatus to detect different types of radioactivity.

**14.** Apparatus according to claim 13, **characterised in that** the semiconductor plate is a monolithic plate comprising one of the following materials: Si, GaAs, CdTe, or CdZnTe.

**15.** Apparatus according to any of claim 10 to 14, **characterised in that** the computer module of the apparatus comprises software which employs the information of the registered vectors of k energy bins for each pixel for determining the contribution from each radioactive marker, in absolute quantities, of the registered energy spectrum for each pixel of the semiconductor sensor plate, by the least squares method, in which a linear combination of the pre-recorded expected energy spectrum for every radioactive marker is fitted to the measured composite energy spectrum, and/or the maximum likelihood method, in which the expected energy spectrum for every radioactive marker is employed to find the most probable number of hits from each radioactive marker in each energy bin which would give the registered energy histogram.

**16.** Apparatus according to any of claim 10 to 15, **characterised in that** the real time data organisation module which keep track and stores the position and energy of every registered radioactive event, and which divides the registered events for each pixel, defined by x,y-position, of the semiconductor detector into a vector of k energy bins, is able to condense the registered and stored data by adding the information from the vectors of all neighbouring pixels into the vector of a centre pixel in order to save computing time, to bring the virtual resolution of the digital map into conformity with the physical planar resolution of probe surface, and/or increase the statistical accuracy of the estimated contributions.

**17.** Apparatus according to claim 16,
**characterised in that** the real time data organisation module can add any number of surrounding rows of pixels to bring the virtual resolution into conformity with coarse arrays on the probe surface, that is, in the case one row is added, all 8 neighbouring pixels that surrounds a centre pixel is added into the centre pixel, in the case when two rows are added, all 8 pixels in the first row and 16 pixels of the second surrounding row are added into the centre pixel, and so forth.

**18.** Apparatus according to any of claim 10 to 17,
**characterised in that** the module for presenting the determined activity of each radioactive label at each test region on the probe surface comprises graphical means for presenting the data in the form of a virtual map representing the probe surface, where each radioactive nuclide is represented by a distinctive colour and where the determined quantity of each radioactive label at each test region of the probe surface is represented by the intensity of the corresponding colour, such that a colour scale is created in the virtual map of the biological array which is directly comparable to the output from the conventional fluorophore technique.

**19.** Apparatus according to any of claim 10 to 17,
**characterised in that** the module for presenting the determined activity of each radioactive nuclide comprises means for presenting the data in the form of histogram maps which depict the quantities of each radioactive nuclide in absolute quantities for each test region of the probe surface.

**20.** Apparatus according to any of claim 10 to 19,
**characterised in that** the apparatus is equipped with means for determining the physical extension by means of the uppermost, lowermost, leftmost and rightmost position of each test spot on the biological array by adding all bands of the energy band images into one single sum image, calculate the x-axis and y-axis projection of the sum image, repeat the process when the image of biological array is rotated in steps of 0.1°, and determine the rotation that aligns the rows of spots with the x-axis from the variance of y-axis projections of the sum images, and that the projections are used to locate the rows and columns of spots in the image.

**Patentansprüche**

**1.** Verfahren für die gleichzeitige Quantifizierung der Mengen von einem oder mehreren radioaktiven Nukliden innerhalb von beliebigen Regionen auf einer Sondenoberfläche, auf der diese Nuklide aufgebracht, adsorbiert oder fixiert worden sind, wobei jedes der radioaktiven Nuklide als Marker an Verbindungen dient, die in Zielgewebeabschnitte, chemische Zielverbindungen oder biologische Zielmoleküle integriert worden sind, die auf chemische oder biologische Sondensubstanzen auf der Sondenoberfläche aufgebracht, auf diesen adsorbiert oder fixiert worden sind,
**dadurch gekennzeichnet,**

- **daß** sowohl die Position als auch die Energie der emittierten Teilchen und/oder Photonen von jeder Region der Sondenoberfläche registriert werden, und
- **daß** die registrierte Information von jeder Region dafür verwendet wird, um die Mengen und/oder Aktivitäten von jedem in dieser Region vorhandenen Radionuklid durch eine beliebige statistische Auswertung der Information in absoluten Mengen zu bestimmen.

**2.** Verfahren nach Anspruch 1,
**dadurch gekennzeichnet,**

- **daß** sowohl die registrierte Position als auch die Energie der emittierten Teilchen und/oder Photonen von jeder Region der Sondenoberfläche als Energieband-Bild, d.h. als Vektor von $k_{max}$ Energieintervallen für jede Region r über die gesamte Sondenoberfläche gespeichert und akkumuliert werden.

**3.** Verfahren nach Anspruch 2,
**dadurch gekennzeichnet,**

- **daß** die statistische Auswertung die Methode der kleinsten Quadrate beinhaltet, wobei eine lineare Kombination der vorab aufgezeichneten Energiespektren für jeden radioaktiven Marker - wobei die unbekannten Koeffizienten von den jeweiligen Radionukliden beigetragen werden - an die Daten von einem Bildelement oder eine Region eines Energieband-Bildes angepaßt wird, die aus einem gemessenen zusammengesetzten Energiehistogramm oder einem Vektor ausgewählt werden, und/oder

- die Maximum-Likelihood-Methode beinhaltet, wobei das gemessene Energiespektrum für jeden radioaktiven Marker dafür verwendet wird, die wahrscheinlichste Anzahl von Treffern von jedem radioaktiven Marker in jedem Energieintervall aufzufinden, die das registrierte Energiehistogramm oder den Vektor ergeben würde, um **dadurch** die Gesamtanzahl von Ereignissen zu ermitteln, die von jedem der Radionuklide ausgehen.

4.  Verfahren nach Anspruch 2 oder 3,
    wobei das Verfahren an biologischen Mikroarrays ausgeführt wird, wobei die Sondenregionen Durchmesser von weniger als 200 $\mu$m aufweisen, oder an Makroarrays ausgeführt wird, wobei die Sondenregionen Durchmesser von mehr als 300 $\mu$m aufweisen.

5.  Verfahren nach Anspruch 1 bis 3,
    **dadurch gekennzeichnet,**
    **daß** das eine oder die mehreren radioaktiven Nuklide entweder Alpha-, Beta-, Gamma- oder Positron-Teilchen und/ oder Photonen emittieren und daß sie derart in Zielmoleküle integriert werden, daß die Radiaktivität auf die Größenordnung von 1 bis 100 Bq von jeder Sondenregion an der Sondenoberfläche abgestimmt wird.

6.  Verfahren nach Anspruch 5,
    **dadurch gekennzeichnet,**
    **daß** es sich bei den radioaktiven Nukliden um nicht-monoenergetische radioaktive Markierungen, einschließlich beta-emittierender Nuklide, handelt, so daß sich partiell und/oder vollständig überlappende Energiespektren ergeben.

7.  Verfahren nach Anspruch 6,
    **dadurch gekennzeichnet,**
    **daß** die beta-emittierenden Radionuklide $^{33}$P and $^{35}$S beinhalten.

8.  Verfahren nach Anspruch 7,
    wobei das Verfahren zum Überwachen der gesamten mRNA-Population in einer Probe von einem Zelltyp / einer Zellreihe durch Vergleich mit einer Probe von einem Standard-Zelltyp / einer Standard-Zellreihe dient, wobei

    - ein a35S-UTP und ein a33P-UTP zu einem Zeitpunkt 0 in geeigneten Konzentrationen zu dem Kulturmedium der parallelen Zellkulturen zugegeben werden,
    - man anschließend die Zellen bis zu einem Zeitpunkt 1 wachsen läßt, wenn die radioaktiven Nukleotide ausreichend integriert sind, um die Detektion von in den Zellen ab dem Zeitpunkt 0 produzierter RNA zu ermöglichen,
    - zu dem Zeitpunkt 1 eine Parallele chemisch behandelt wird, um eine RNA-Synthese zu blockieren, und
    - zu einem Zeitpunkt 2 beide Kulturen geerntet werden, RNA isoliert wird und gleiche Mengen von RNA von beiden Kulturen gemischt werden und schließlich
    - die Mischung als kombinierte cDNA-Sonde behandelt wird und auf ein biologisches Mikroarray hybridisiert wird, von dem die Gesamtmengen der beiden radioaktiven Marker bestimmt werden können.

9.  Verfahren nach einem der Ansprüche 1 bis 7,
    wobei das Verfahren zum gleichzeitigen Überwachen von verschiedenen Sätzen von Protein-Phosphorylierungsgraden dient, indem radioaktiv markierte Aminosäure-Tags in das Medium integriert werden und diese anschließend auf biologischen Mikroarrays adsorbiert werden, die strukturierte Sätze von Antikörpern enthalten.

10. Vorrichtung zum Ausführen einer gleichzeitigen Quantifizierung der Mengen von einem oder mehreren radioaktiven Nukliden innerhalb von beliebigen Testregionen auf einer Sondenoberfläche, auf die diese Nuklide aufgebracht, adsorbiert oder fixiert worden sind,
    **dadurch gekennzeichnet,**
    **daß** die Vorrichtung folgendes aufweist:

    - ein elektronisches System für Echtzeit-Messungen von Trefferpositionen und aufgebrachten Energien von radioaktiven Teilchen/Photonen, die von den Testregionen der Sondenoberfläche emittiert werden, und das folgendes aufweist: einen mehrstreifigen Halbleiterdetektor mit orthogonalen p-leitenden Streifen bzw. n-leitenden Streifen auf gegenüberliegenden Seiten sowie eine Ausleseelektronik, die zum Detektieren der durch die X-, Y-Koordinate definierten Trefferposition an dem Detektor für jedes radioaktive Ereignis sowie der aufgebrachten Energie des Teilchens/Photons in der Lage ist, einen Lademechanismus, der ein Positionieren der Sondenoberfläche in Berührung mit dem Halbleiterdetektor über ihre gesamte Oberfläche ermöglicht, und zwar getrennt lediglich durch eine dünne Mylarfolie zum Schutz des Halbleiterdetektors,

- ein Echtzeit-Datenorganisationsmodul, das die Position und Energie von jedem registrierten radioaktiven Ereignis verfolgt und speichert und das die registrierten Ereignisse für jedes durch die X-, Y-Position definierte Pixel des Halbleiterdetektors in einen Vektor von k Energieintervallen unterteilt und **dadurch** ein Energieband-Bild bildet,

- ein Computer-Modul mit Hardware und Software, das in der Lage ist, das Ernergieband-Bild für die Bestimmung der Mengen oder der Aktivität von jeder an einem beliebigen Pixel oder einer kondensierten Region der Halbleitersensorplatte vorhandenen radioaktiven Markierung in absoluten Mengen zu verwenden, und

- ein Modul zum Anzeigen der Verteilung der Aktivität von jedem radioaktiven Marker über jedem Pixel der Halbleiterplatte oder wahlweise in ausgewählten Testregionen an der Sondenoberfläche.

**11.** Vorrichtung nach Anspruch 10,
**dadurch gekennzeichnet,**
**daß** die Ausleseelektronik für Echtzeit-Messungen von Trefferpositionen und aufgebrachten Energien von radioaktiven Teilchen/Photonen folgendes aufweist:

- einen Verstärkerkanal, bestehend aus einer Vorverstärker- und Filtereinheit, und eine Auslöseeinheit und Abtast- und Halteeinheit parallel für jeden Streifen, und
- ein Datenerfassungssystem in Verbindung mit den Verstärkerkanälen, das in der Lage ist, die Position (X, Y) und die Energie von allen registrierten radioaktiven Ereignissen zu verfolgen, die auf den sensitiven Teil der Halbleitersensorplatte auftreffen.

**12.** Vorrichtung nach Anspruch 10 oder 11,
**dadurch gekennzeichnet,**
**daß** der Halbleiterdetektor eine quadratische Platte aus einem monolithischen Halbleitermaterial mit typischen Abmessungen von zehn Quadratzentimetern und einer Dicke von ca. 300 $\mu$m und einer sensitiven Fläche von 64 x 32 mm$^2$ aufweist, wobei die Streifen-Mittenbeabstandung der sensitiven n-leitenden Bänder und der orthogonal orientierten p-leitenden Streifen auf der gegenüberliegenden Seite der Halbleiterplatte 50 $\mu$m beträgt, so daß sich insgesamt 1280 x 640 Streifen oder ein zweidimensionales Gitter von 820000 Pixeln ergeben.

**13.** Vorrichtung nach Anspruch 10 bis 12,
**dadurch gekennzeichnet,**
**daß** der Lademechanismus und der Halbleiterdetektor derart ausgebildet sind, daß die Halbleiterplatte entfernt und durch eine andere Halbleiterplatte mit ähnlicher Pixelausbildung und ähnlichen Abmessungen, jedoch aus einem anderen Halbleitermaterial, ersetzt werden kann, so daß die Vorrichtung verschiedene Arten von Radioaktivität detektieren kann.

**14.** Vorrichtung nach Anspruch 13,
**dadurch gekennzeichnet,**
**daß** es sich bei der Halbleiterplatte um eine monolithische Platte handelt, die eines der folgenden Materialien aufweist: Si, GaAs, CdTe oder CdZnTe.

**15.** Vorrichtung nach einem der Ansprüche 10 bis 14,
**dadurch gekennzeichnet,**
**daß** das Computermodul der Vorrichtung Software aufweist, die die Information von den registrierten Vektoren von k Energieintervallen für jedes Pixel zum, in absoluten Mengen, erfolgenden Bestimmen des Beitrags von jedem radioaktiven Marker des registrierten Energiespektrums für jedes Pixel der Halbleitersensorplatte durch die Methode der kleinsten Quadrate verwendet, wobei eine lineare Kombination des vorab aufgezeichneten erwarteten Energiespektrums für jeden radioaktiven Marker an das gemessene zusammengesetzte Energiespektrum angepaßt wird, und/oder die Maximum-Likelihood-Methode verwendet, bei der das erwartete Energiespektrum für jeden radioaktiven Marker zum Auffinden der wahrscheinlichsten Anzahl von Treffern von jedem radioaktiven Marker in jedem Energieintervall verwendet wird, die zu dem registrierten Energiehistogramm führen würde.

**16.** Vorrichtung nach einem der Ansprüche 10 bis 15,
**dadurch gekennzeichnet,**
**daß** das Echtzeit-Datenorganisationsmodul, das die Position und die Energie von jedem registrierten radioaktiven Ereignis verfolgt und speichert und das die registrierten Ereignisse für jedes durch die X-, Y-Position definierte Pixel des Halbleiterdetektors in einen Vektor von k Energieintervallen unterteilt, in der Lage ist, die registrierten und gespeicherten Daten zu kondensieren, indem die Information von den Vektoren von allen benachbarten Pixeln in den Vektor eines zentralen Pixels addiert wird, um **dadurch** Rechenzeit einzusparen, um die virtuelle Auflösung der digitalen Karte in Übereinstimmung mit der physikalischen planaren Auflösung der Sondenoberfläche zu bringen und/oder die statistische Genauigkeit der geschätzten Beiträge zu erhöhen.

**17.** Vorrichtung nach Anspruch 16,
**dadurch gekennzeichnet,**
**daß** das Echtzeit-Datenorganisationsmodul eine beliebige Anzahl von umgebenden Pixelreihen addieren kann, um die virtuelle Auflösung in Übereinstimmung mit groben Arrays auf den Sondenoberfläche zu bringen, d.h. bei der Addition von einer Reihe werden alle 8 benachbarten Pixel, die ein zentrales Pixel umgeben, zu dem zentralen Pixel addiert, bei der Addition von zwei Reihen werden alle 8 Pixel in der ersten Reihe sowie 16 Pixel der zweiten umgebenden Reihe zu dem zentralen Pixel addiert, usw.

**18.** Vorrichtung nach einem der Ansprüche 10 bis 17,
**dadurch gekennzeichnet,**
**daß** das Modul zum Präsentieren der festgestellten Aktivität von jeder radioaktiven Markierung in jeder Testregion an der Sondenoberfläche graphische Einrichtungen zum Präsentieren der Daten in Form einer die Sondenoberfläche darstellenden virtuellen Karte aufweist, wobei jedes radioaktive Nuklid durch eine separate Farbe dargestellt wird und wobei die festgestellte Menge von jeder radioaktiven Markierung in jeder Testregion der Sondenoberfläche durch die Intensität der entsprechenden Farbe dargestellt wird, so daß eine Farbskala in der virtuellen Karte des biologischen Array erzeugt wird, die mit dem Ausgabe von der herkömmlichen Fluorophor-Technik direkt vergleichbar ist.

**19.** Vorrichtung nach einem der Ansprüche 10 bis 17,
**dadurch gekennzeichnet,**
**daß** das Modul zum Präsentieren der festgestellten Aktivität jedes radioaktiven Nuklids Einrichtungen zum Präsentieren der Daten in Form von Histogrammkarten aufweist, die die Mengen von jedem radioaktiven Nuklid in absoluten Mengen für jede Testregion der Sondenoberfläche darstellen.

**20.** Vorrichtung nach einem der Ansprüche 10 bis 19,
**dadurch gekennzeichnet,**
**daß** die Vorrichtung ausgestattet ist mit Einrichtungen zum Bestimmen der physischen Erstreckung anhand der obersten, untersten, äußersten linken und äußersten rechten Position von jedem Testpunkt auf dem biologischen Array durch Addieren von allen Bändern der Energieband-Bilder zu einem einigen Summen-Bild, Berechnen der X-Achsen- und der Y-Achsen-Projektion des Summen-Bildes, Wiederholen des Vorgangs bei rotationsmäßiger Bewegung des Bildes des biologischen Arrays in Schritten von 0,1° und Bestimmen der Rotation, bei der die Reihen der Punkte ausgehend von der Varianz der Y-Achsen-Projektionen der Summen-Bilder mit der X-Achse ausgerichtet werden,
und **daß** die Projektionen zum Lokalisieren der Reihen und Spalten von Punkten in dem Bild verwendet werden.

## Revendications

**1.** Méthode de quantification simultanée des quantités d'un ou de plusieurs radionucléides à l'intérieur de régions arbitraires se trouvant sur la surface d'une sonde où ces nucléides ont été déposés, adsorbés ou fixé, dans laquelle chaque nucléide radioactif sert de marqueurs sur des composés qui ont été incorporés dans des sections de tissus cibles, des composés chimiques cibles ou des molécules biologiques cibles qui ont été déposés, adsorbés ou fixés sur des substances sondes chimiques ou biologiques sur la surface de la sonde, **caractérisée en ce que**

✔ la position et l'énergie des particules émises et/ou des photons émis par chaque région de la surface de la sonde sont toutes deux enregistrées, et
✔ les informations enregistrées provenant de chaque zone sont utilisées pour déterminer, par des grandeurs absolues, les quantités et/ou les activités de chaque radionucléide présent dans cette région, grâce à des évaluations statistiques appropriées des informations.

2. Méthode selon la revendication 1, **caractérisée en ce que** la position et l'énergie enregistrées des particules émises et/ou des photons émis par chaque région de la surface de la sonde sont toutes deux stockées et accumulées sous la forme d'une image de bandes d'énergie, c'est-à-dire stockées sous la forme d'un vecteur de tranches d'énergie $k_{max}$ pour chaque région r sur la totalité de la surface de la sonde.

3. Méthode selon la revendication 2, **caractérisée en ce que** l'évaluation statistique comprend la méthode des moindres carrés, dans laquelle une combinaison linéaire des spectres d'énergie préenregistrés de tous les marqueurs radioactifs - les coefficients inconnus étant les contributions provenant de chacun des radionucléides - est ajustée aux données issues d'un élément d'image ou d'une région d'une image de bandes d'énergie choisi parmi un histogramme d'énergie composite mesuré ou un vecteur, et/ou la méthode du maximum de vraisemblance, moyennant quoi le spectre d'énergie mesuré de chaque marqueur radioactif est utilisé pour trouver le nombre le plus probable de coups provenant de chaque marqueur radioactif dans chaque tranche d'énergie qui donnerait l'histogramme d'énergie enregistré ou le vecteur, pour ainsi fournir le nombre total d'événements provenant de chacun des radionucléides.

4. Méthode selon la revendication 2 ou 3, dans laquelle la méthode est mise en oeuvre sur des micropuces biologiques où les régions de sonde ont des diamètres inférieurs à 200 $\mu$m, ou sur des macropuces où les régions de sonde ont des diamètres supérieurs à 300 $\mu$m.

5. Méthode selon les revendications 1 à 3, **caractérisée en ce que** le ou les radionucléides émettent comme particules des particules alpha, bêta, gamma ou des positons et/ou des photons, et **en ce qu'**ils sont incorporés dans des molécules cibles de manière à ce que la radioactivité provenant de chaque région de sonde se trouvant sur la surface de la sonde soit de l'ordre de 1 Bq à 100 Bq.

6. Méthode selon la revendication 5, **caractérisée en ce que** les radionucléides sont des traceurs radioactifs non monoénergétiques, notamment des nucléides à émission bêta, donnant des spectres d'énergie se chevauchant partiellement et/ou totalement.

7. Méthode selon la revendication 6, **caractérisée en ce que** les radionucléides à émission bêta comprennent $^{33}$P et [35]S.

8. Méthode selon la revendication 7, dans laquelle la méthode sert à surveiller la totalité d'une population d'ARNm se trouvant dans un échantillon provenant d'un type de cellule/d'une lignée cellulaire en effectuant une comparaison avec un échantillon provenant d'un type de cellule étalon/d'une lignée cellulaire étalon, où

✓ du a35S-UTP et du a33P-UTP sont ajoutés au milieu de culture de cultures parallèles de cellules à des concentrations appropriées au temps 0,
✓ ensuite, les cellules sont laissées croître jusqu'au temps 1, quand les nucléotides radioactifs sont suffisamment incorporés pour permettre la détection de l'ARN produit dans les cellules à partir du temps 0,
✓ au temps 1, une culture parallèle est traitée par voie chimique pour bloquer la synthèse de l'ARN, et
✓ au temps 2, les deux cultures sont collectées, l'ARN est isolé et des quantités identiques d'ARN provenant des deux cultures sont mélangées, et finalement
✓ le mélange est traité sous la forme d'une sonde combinée d'ADNc et hybridé à une micropuce biologique, à partir duquel les quantités totales des deux marqueurs radioactifs peuvent être déterminées.

9. Méthode selon l'une quelconque des revendications 1 à 7, dans laquelle la méthode sert à suivre simultanément divers ensembles de degrés de phosphorylation de protéines par l'incorporation d'étiquettes de type acide aminé radiomarqué dans le milieu puis par leur adsorption sur des micropuces biologiques contenant des ensembles structurés d'anticorps.

10. Appareil permettant d'effectuer la quantification simultanée des quantités d'un ou de plusieurs nucléides radioactifs se trouvant dans des régions d'essai arbitraires sur la surface d'une sonde où ces nucléides ont été déposés, adsorbés ou fixés, **caractérisé en ce que** l'appareil comprend

✓ un système électronique permettant la mesure en temps réel des positions des coups et des énergies déposées des particules radioactives/ photons qui sont émis par les régions d'essai de la surface de la sonde, et qui comprend un détecteur à semi-conducteur multi-bandes possédant respectivement des bandes orthogonales de type p et de type n sur des côtés opposés, et de l'électronique de lecture qui est capable de détecter la position des coups, définie par des coordonnées x et y, sur le détecteur pour chaque événement radioactif ainsi que l'énergie déposée par la particule/le photon, un mécanisme de chargement permettant à la surface de la sonde d'être placée au contact du détecteur à semi-conducteur sur la totalité de sa surface, séparée seulement par une fine feuille de mylar pour protéger le détecteur à semi-conducteur,

✓ un module d'organisation en temps réel des données qui conserve la trace et qui stocke la position et l'énergie de chaque événement radioactif enregistré, et qui divise les événements enregistrés pour chaque pixel, défini par une position x,y, du détecteur à semi-conducteur en vecteur de tranches d'énergie k, pour ainsi former une image de bandes d'énergie,

✓ un module informatique comprenant un matériel et un logiciel capables d'utiliser l'image de bandes d'image pour déterminer, par des grandeurs absolues, les quantités ou l'activité de chaque traceur radioactif présent sur l'un quelconque des pixels ou la région condensée de la plaque de détection à semi-conducteur, et

✓ un module pour afficher la distribution de l'activité de chaque marqueur radioactif à travers chaque pixel de la plaque à semi-conducteur ou, facultativement, dans des régions d'essai sélectionnées sur la surface de la sonde.

**11.** Appareil selon la revendication 10, **caractérisé en ce que** l'électronique de lecture permettant une mesure en temps réel des positions des coups et des énergies déposées des particules radioactives/des photons comprend

✓ un canal amplificateur constitué d'une unité de pré-amplification et de filtrage, et d'une unité de déclenchement et d'une unité d'échantillonnage/blocage en parallèle pour chaque bande, et

✓ un système d'acquisition de données en communication avec les canaux amplificateurs et qui est capable de conserver la trace de la position, définie par x,y, et l'énergie de tous les événements radioactifs enregistrés qui frappent la partie sensible de la plaque de détection à semi-conducteur.

**12.** Appareil selon la revendication 10 ou 11, **caractérisé en ce que** le détecteur à semi-conducteur comprend une plaque quadratique composée d'un matériau semi-conducteur monolithique de dix centimètres carrés ayant une épaisseur d'environ 300 $\mu$m et qui a une zone sensible de 64 X 32 mm$^2$, et où le pas de bande entre les bandes sensibles de type n et les bandes de types p orientées de manière orthogonale sur le côté opposé de la plaque à semi-conducteur est de 50 $\mu$m, ce qui donne au total 1280 x 640 bandes ou une grille bidimensionnelle de 820 000 pixels

**13.** Appareil selon les revendications 10 à 12, **caractérisé en ce que** le mécanisme de chargement et le détecteur à semi-conducteur sont conçus de manière à ce que la plaque à semi-conducteur puisse être enlevée et remplacée par une autre plaque à semi-conducteur ayant une conception de pixel identique et des dimensions identiques, mais composée d'un autre matériau semi-conducteur pour permettre à l'appareil de détecter différents types de radioactivité.

**14.** Appareil selon la revendication 13, **caractérisé en ce que** la plaque à semi-conducteur est une plaque monolithique comprenant l'un des matériaux suivants : Si, GaAs, CdTe ou CdZnTe.

**15.** Appareil selon l'une quelconque des revendications 10 à 14, **caractérisé en ce que** le module informatique de l'appareil comprend un logiciel qui utilise les informations des vecteurs de tranches d'énergie k enregistrés pour chaque pixel afin de déterminer la contribution issue de chaque marqueur radioactif, en quantité absolue, du spectre d'énergie enregistré pour chaque pixel de la plaque de détection à semi-conducteur, par la méthode des moindres carrés, dans laquelle une combinaison linéaire des spectres d'énergie attendus pré-enregistrés de tous les marqueurs radioactifs est ajustée au spectre d'énergie composite mesuré, et/ou par la méthode du maximum de vraisemblance, dans laquelle le spectre d'énergie attendu de chaque marqueur radioactif est utilisé pour trouver le nombre le plus probable de coups provenant de chaque marqueur radioactif dans chaque tranche d'énergie qui donnerait l'histogramme d'énergie enregistré.

**16.** Appareil selon l'une quelconque des revendications 10 à 15, **caractérisé en ce que** le module d'organisation en temps réel des données qui suit et stocke la position et l'énergie de chaque événement radioactif enregistré, et qui divise les événements enregistrés pour chaque pixel, défini par une position x,y, du détecteur à semi-conducteur en vecteur de tranches d'énergie k, est capable de condenser les données enregistrées et stockées en ajoutant les informations provenant des vecteurs de tous les pixels voisins dans le vecteur d'un pixel central afin de gagner du temps de calcul, de mettre la résolution virtuelle de la carte numérique en conformité avec la résolution plane physique de la surface de la sonde et/ou d'augmenter la précision statistique des contributions estimées.

**17.** Appareil selon la revendication 16, **caractérisé en ce que** le module d'organisation en temps réel des données peut ajouter un certain nombre de rangées de pixels environnantes pour mettre la résolution virtuelle en conformité avec les réseaux grossiers de la surface de la sonde, c'est-à-dire que quand une rangée est ajoutée, les 8 pixels voisins qui entourent un pixel central sont tous ajoutés dans le pixel central, quand deux rangées sont ajoutées, les 8 pixels de la première rangée et les 16 pixels de la seconde rangée environnante sont tous ajoutés dans le pixel central, et ainsi de suite.

**18.** Appareil selon l'une quelconque des revendications 10 à 17, **caractérisé en ce que** le module servant à présenter l'activité déterminée de chaque traceur radioactif dans chaque région d'essai se trouvant sur la surface de la sonde comprend des moyens graphiques pour présenter les données sous la forme d'une carte virtuelle représentant la surface de la sonde, où chaque nucléide radioactif est représenté par une couleur distincte et où la quantité déterminée de chaque traceur radioactif dans chaque région d'essai de la surface de la sonde est représentée par l'intensité de la couleur correspondante, de sorte qu'une échelle de couleurs est créée sur la carte virtuelle de la puce biologique qui peut être directement comparée aux résultats obtenus avec la technique classique au fluorophore.

**19.** Appareil selon l'une quelconque des revendications 10 à 17, **caractérisé en ce que** le module servant à présenter l'activité déterminée de chaque radionucléide comprend des moyens pour présenter les données sous la forme de cartes d'histogramme qui représentent les quantités de chaque radionucléide en quantités absolues pour chaque région d'essai de la surface de la sonde.

**20.** Appareil selon l'une quelconque des revendications 10 à 19, **caractérisé en ce que** l'appareil est équipé de moyens permettant de déterminer l'extension physique au moyen de la position la plus haute, la plus basse, la plus à gauche et la plus à droite de chaque spot d'essai se trouvant sur la puce biologique par l'addition de toutes les bandes des images de bandes d'énergie dans une unique image globale, le calcul de la projection sur l'axe des x et l'axe des y de l'image globale, la répétition du procédé lors de la rotation de l'image de la puce biologique par pas de 0,1°, et la détermination de la rotation qui aligne les rangées de spots avec l'axe des x à partir de la variance des projections sur l'axe des y des images globales, et **en ce que** les projections sont utilisées pour localiser les rangées et les colonnes de spots sur l'image.

2

N+ strips

P+ strips

Figure 1a

Figure 1b

amplitude

trigger

One Amplifier Channel

S/H

Trigg

preamplifier & filter

From strip

Figure 1c

Figure 1d

Fig. 2

EP 1 358 357 B1

Virtual map representation & screen image

Hardware          Software

26

FIG.3

$$E(x,y,E1) = E(x,y,E1)+1$$

X,Y

E1

E2

E3

X,Y,E

X,Y
E+ΔE

E19

E20

Energy
Correct.
Data.
ΔE

Spectra
α(k) and β(k) for the
two Radionuclides

Energy band
images

E(x,y,k)

Condens.
Energy b.
Images

C(i,j,k)

Minimize
$$\Sigma(A(i,j)*\alpha(k) + B(i,j)*\beta(k) - CE(i,j,k))^2$$

A(i,j)

B(i,j)

Spectra of $^{35}S$ and $^{33}P$

FIG. 4

Projection on x-axis

10⁶
10⁵
10⁴
10³
10²
10¹
10⁰

10⁻³  10⁻¹    ~1 Bequerel/spot
10⁻⁴  10⁻²

400µM

250µM

33P

Carry-over
contamination

35S

33P + 35S

FIG.5

FIG. 6

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- GB 2311198 A
- US 5656818 A
- EP 0983705 A

### Non-patent literature cited in the description

- **Wang, E. ; Miller, L.D. ; Ohnmacht, G.A. ; Liu, E.T.** High-fidelity mRNA amplification for gene profiling. *Marincola FM Nat Biotechnol,* 2000, vol. 18 (4), 457-459
- **Nilsen, T. W. ; Grayzel, J. ; Prensky, W.** Dendritic Nucleic Acid Structures. *J. theor. Biol.,* 1997, vol. 187, 273-284
- **Wang, J. ; Rivas, G. ; Fernandes, J. R. ; Jiang, M. ; Paz, J. L. L. ; Waymire, R. ; Nilsen, T. W. ; Getts, R. C.** Adsorbtion and Detection of DNA Dendrimers at Carbon Electrodes. *Electroanalysis,* 1998, vol. 10, 553-556
- **Wang, J. ; Jiang, M. ; Nilsen, T. W. ; Getts, R. C.** Dendritic Nucleic Acid Probes for DNA Biosensors. *J. Am. Chem. Soc.,* 1998, vol. 120, 8281-8282
- **Kvinnsland, Y. ; Skretting, A.** Methods for separation of contributions from two radionuclides in autoradiography with a silicon strip detector. *Phys.Med.Biol.,* 2000, vol. 45, 1183-1193